(19) Europäisches Patentamt

European Patent Office

Office européen des brevets

(11) Publication number: **0 453 719 B1**

## (12) EUROPEAN PATENT SPECIFICATION

(45) Date of publication of patent specification: **28.12.94**　(51) Int. Cl.⁵: **C07K 7/10**, C12P 21/02

(21) Application number: **91101895.0**

(22) Date of filing: **11.02.91**

54 **Bacteriocin peptide.**

(30) Priority: **25.04.90 US 514102**

(43) Date of publication of application:
**30.10.91 Bulletin 91/44**

(45) Publication of the grant of the patent:
**28.12.94 Bulletin 94/52**

(84) Designated Contracting States:
**AT BE CH DE DK ES FR GB GR IT LI LU NL SE**

56 References cited:
**EP-A- 0 293 547**

(73) Proprietor: **MICROLIFE TECHNICS, INC.**
**1833 57th Street**
**Sarasota,**
**Florida 34230 (US)**

(72) Inventor: **Henderson, James T.**
**6729 5th Street, W.**
**Bradenton,**
**Florida 34207 (US)**
Inventor: **van Wassenaar, Pieter Dirk**
**Churchill Dreef 5**
**NL-3145 BB Maassluis (NL)**

(74) Representative: **Patentanwälte Grünecker,**
**Kinkeldey, Stockmair & Partner**
**Maximilianstrasse 58**
**D-80538 München (DE)**

**EP 0 453 719 B1**

**Description**

The present invention relates to a peptide which is a bacteriocin. In particular, the present invention relates to the peptide in chemically pure form having a specific identified amino acid sequence and which is derived from a bacteriocin isolated from Pediococcus acidilactici NRRL-B-18050.

Prior Art

Bhunia, et al (Bhunia, A. K., et al, J. Indust. Micro. 2 319-322 (1987); and Bhunia, A. K., et al., J. Appl. Bact. 65 261-268 (1988)) reported that Pediococcus acidilactici strain H produced a bacteriocin, pediocin H with a molecular weight estimated by SDS-PAGE to be about 2700 Da (Bhunia, 1988). The reported spectrum of activity, however, includes Staphylococcus aureus which is not a characteristic of the bacteriocin of the present invention.

Tagg et al classify bacteriocins (Tagg, J. R., et al, Bacteriol. Rev. 40, 722-756 (1976)). The lantibiotics are a category of bacteriocins of gram-positive bacteria. They are characterized by the presence of lanthionine bridges and dehydroalanine residues. They are also small, basic proteins. The bacteriocin of the present invention isolated from culture medium shows no evidence of post-translational modification of any of the amino acid side chains and therefore cannot be classified as a lantibiotic although the size and charge are characteristic of lantibiotic proteins. Also the amino acid sequence of some of the lantibiotics is known and is not similar to the bacteriocin of the present invention.

Hoover, et al (Hoover, D. G., et al., J. Food Prot. 51 29-31, (1988)) reported bacterocin activity associated with a 5.5 MDa plasmid in several strains of Pediococcus isolated from fermented sausage including Pediococcus acidilactici NRRL-B-5627 which is essentially the same strain as NRRL-B-18050 described hereinafter. Graham and McKay (Graham, D. C. et al., Appl. Env. Micro. 50, 532-534, (1985)) linked bacteriocin production to a 10.5 MDa plasmid in Pediococcus pentosaceus FBB-63. Daeschel and Klaenhammer (Daeschel, M.A. and Klaenhammer, T. R., Appl. Env. Micro. 50, 1538-1541, (1985)) reported that bacteriocin production in Pediococcus pentosaceus FBB-61 and L7230 was encoded by a 13.6 MDa plasmid. None of the bacteriocins were purified and sequenced. Purification is difficult since the bacteriocins are sensitive to structural changes caused by the purification.

Bacteriocins from Pediococcus acidilactici NRRL-B-18050 is known as described in U.S. Patent No. 4,883,673 to Gonzalez. The bacteriocin produced was an impure form in a growth medium or derived from the growth medium by ammonium sulfate precipitation. The purified bacteriocin had an activity of up to about 32,000 Au per ml. This bacteriocin was determined not to be pure. Also it was determined that the bacteriocin had an apparent molecular weight of about 16,500 as described in U.S patent application Serial No. 07/148,044 filed January 25, 1988 by Vandenbergh. The problem was that since the bacteriocin was still impure it could not be synthesized by synthetic chemical means. The presence of the impurities produced variable results in use of impure bacteriocin.

OBJECTS

It is therefore an object of the present invention to provide a pure, chemically identified bacteriocin which is a peptide. Further, it is an object of the present invention to provide a peptide which can be derived by chemical synthesis. These and other objects will become increasingly apparent by reference to the following description and the drawings.

IN THE DRAWINGS

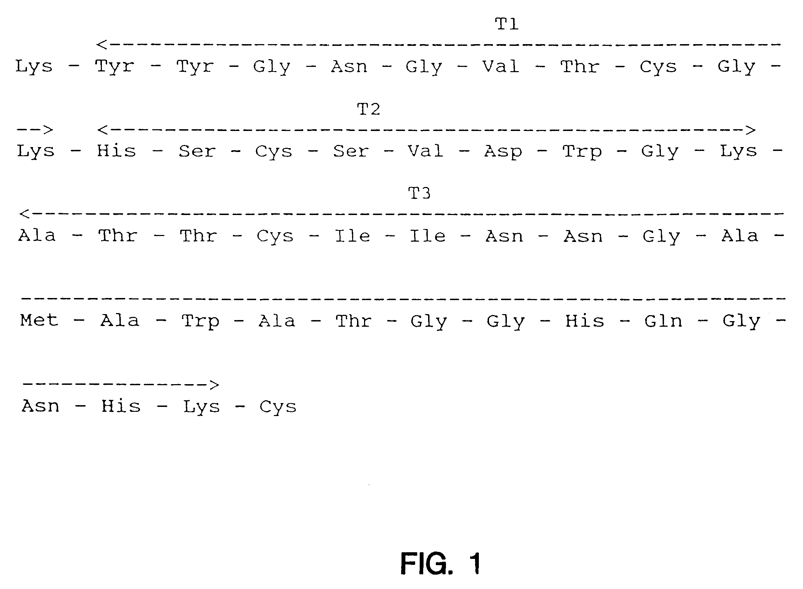

Figure 1 shows the amino acid sequence of the tryptic fragments of the peptide of the present invention.

2

GENERAL DESCRIPTION

The present invention relates to a peptide consisting of an amino acid sequence

```
Lys - Tyr - Tyr - Gly - Asn - Gly - Val - Thr - Cys - Gly -
 1     2     3     4     5     6     7     8     9     10

Lys - His - Ser - Cys - Ser - Val - Asp - Trp - Gly - Lys -
11    12    13    14    15    16    17    18    19    20

Ala - Thr - Thr - Cys - Ile - Ile - Asn - Asn - Gly - Ala -
21    22    23    24    25    26    27    28    29    30

Met - Ala - Trp - Ala - Thr - Gly - Gly - His - Gln - Gly -
31    32    33    34    35    36    37    38    39    40

Asn - His - Lys - Cys
41    42    43    44
```

and disulfide bonds between Cys 9 and Cys 14 and between Cys 24 and Cys 44.

The peptide of the present invention has a molecular weight of 4629 daltons which is about 1/3rd the apparent molecular weight of the impure bacteriocin as it is derived from the growth medium. The bacteriocin exhibits an Au of about 50,000 Au per ml, which is significantly higher than the Au of the impure bacteriocin.

The peptide of the present invention can be synthesized by chemical means. Solid phase peptide synthesis (SPPS) is a method of preparing peptides by chemically coupling the individual amino acids in a specified sequence. It is described in Chem. Anal. Solid Phase Biochem; Chapter 101, pages 507-534 (1983). Synthesis begins at the C-terminal amino acid which is covalently attached to a solid support. The next amino acid is activated at the C-terminal and is blocked from reactivity at the N-terminal; therefore covalent coupling (formation of a peptide bond) occurs between the C-terminal of the second amino acid and the N-terminal of the first amino acid. After coupling is complete, the N-terminal of the second amino acid is deprotected and the cycle is begun again with the third amino acid. After addition of the final amino acid (the N-terminal of the peptide) the peptide is cleaved from the solid support and the various side chain protecting groups are removed.

In the original Merrifield method of SPPS, the solid support is typically a chloromethylated copolymer of styrene and divinylbenzene. Cleavage from this support gives a free acid group at the C-terminus. For the production of peptide amides, a 4-methyl-benzylhydramine resin is used. N-terminal protection during coupling is provided by a t-butoxycarbonyl (tBOC) group. The protecting group is removed with anhydrous hydrochloric acid and the resulting amine hydrochloride is neutralized with triethylamine. The next t-BOC amino acid is activated with dicyclohexylcarbodiimide or diisopropylcarbodiimide and coupled to the prior amino acid. The cycle is repeated for each subsequent amino acid. Peptide-resin cleavage and side chain deprotection is performed simultaneously with anhydrous hydrofluoric acid to give the peptide or peptide amide.

Improvements to the Merrifield method deal with solutions to specific problems which may result in reduced yield. Insufficient yield is a primary limiting factor in the production of long sequences. An example of such an improvement is the use of the 9-fluorenylmethoxycarbonyl(Fmoc) protecting group. The Fmoc protectant provides an acid stable group which is removed by treatment with an amine such as piperidine. The use of Fmoc can improve yield since it provides for the use of base for deprotection rather than acid. The use of acid deprotectant in the original Merrifield procedure has been associated with premature cleavage of the peptide-resin bond resulting in lowered yield.

SPECIFIC DESCRIPTION

The bacteriocin PA-1 was purified to homogeneity by gel filtration and ion exchange chromatography, and reversed-phase liquid chromatography after considerable effort and numerous failures. The amino acid sequence of PA-1 was found to correspond to an open reading frame of the DNA sequence of a 6.2 megaldalton plasmid associated with bacteriocin activity in Pediococcus acidilactici PAC 1.0. From the sequence data, the conclusion was made that PA-1 was a basic protein with a molecular mass of 4629 daltons. The presence of four cysteine residues gave rise to the possibility of two disulfide bridges. Analysis of tryptic fragments showed that a disulfide bond existed between Cys-9 and Cys-14 and between Cys-24 and Cys-44. The purified bacteriocin was active against the bacterium, Listeria monocytogenes and other bacteria as set forth hereinafter.

Example 1

Growth Medium, Bacteriocin Production, And Assay Of Activity. One liter of MRS broth (Difco, Detroit, Michigan) with 4% yeast extract (Oxoid™, Basingstoke, England) was inoculated at 1% by volume with an 8 hour old culture of strain PAC 1.0 grown in MRS broth and was grown statically at 35°C for 18 hours. Cells were removed by centrifugation at 16,300 x g for 15 minutes at 4°C. The supernatant was filtered through a 0.20 micron filter (Millipore) and was kept frozen at -20°C until use. Bacteriocin production was assayed by spotting 5 ul of a serial twofold dilution series onto MRS plates overlaid with soft agar seeded with Pediococcus pentosaceus FBB63 indicator cells. One AU is defined as the highest dilution yielding a definite zone on the indicator lawn.

Purification of PA-1. Supernatant was neutralized to pH 6.0 with sodium hydroxide prior to gel filtration. A 450 ml aliquot of neutralized supernatant was applied to a 5 cm x 55 cm column of Spectra/Gel AcA 202™ (Spectrum, Los Angeles, CA) gel filtration resin which had been equilibrated against 0.05 M 2-(N-morpholino)ethanesulfonic acid (MES) (Research Organics, Cleveland, OH), pH 6.0. Activity was eluted using the same buffer. Activity was obtained beginning at the void volume and continued to elute throughout six void volumes. Active fractions were pooled and applied to a 2.5 cm x 90 cm CM-Sepharose™ column (Pharmacia, Piscataway, NJ) re-equilibrated against 0.05 M MES, PH 6.0. Activity was eluted with a linear gradient to 0.05 M MES containing 1 M sodium chloride, pH 6.0. Active fractions were pooled and dialyzed against a 10 fold excess of water using 1000 Da molecular weight cut-off dialysis tubing (Spectra-Por 6™, Spectrum, Los Angeles, CA). Dialysate volume was reduced 12 fold by applying the dialysis tubing directly to solid 20 KDa polyethylene glycol (Carbowax, Union Carbide) and was then further reduced 3.5 fold by vacuum centrifugation (Speed-Vac™, Savant, Farmingdale, NY). Concentrated PA-1 was applied to a 1.0cm x 25cm C18 reversed-phase column (Vydac™, Hesperia, CA) equilibrated with 0.1% trifluoroacetic acid. Activity was eluted with a linear gradient to 45% acetonitrile over 30 minutes at 1.5 ml/min. Active fractions were determined by directly spotting aliquots of column effluent on MRS plates overlaid with soft agar containing Pediococcus pentosaceus FBB63 cells. Active fractions were dried by vacuum centrifugation and stored at -20°C.

Example 2

Analysis of Purified Bacteriocin

Amino Acid Analysis. Samples for hydrolysis were dried by vacuum dehydration, reconstituted in 0.1 ml 6N HCl, dried again, and hydrolyzed in vacuo at 100 deg C in 8mm x 60mm hydrolysis tubes (Pierce, Rockford, IL) for 24 hours in a Reacti-Therm™ heating block (Pierce, Rockford, IL). Phenyl isothiocyanate (PITC) derivatives were made using the directions supplied with a Pico-Tag derivatization system (Waters, division of Millipore, Milford, MA) and the PITC derivatives were detected at 254 nm using the suggested Pico-Tag protocol except that the reversed-phase column was a 0.45cm x 25cm C18 column (Vydac, Hesperia, CA) instead of the proprietary Pico-Tag column.

Amino Acid Sequencing. Sequence information was obtained with a gas phase sequencer ABI 475A using protocols supplied by ABI (Applied Biosystems, Inc., Foster City, California). To obtain the sequence of the C terminal region, the protein was first cleaved at Met-31 by mising with a 2 mg/ml solution of cyanogen bromide in 88% formic acid overnight at room temperature. Sequence information was then obtained simultaneously beginning with residues 1 and 32.

SDS Polyacrylamide Gel Electrophoresis. Gels were prepared at either a fixed acrylamide concentration of 12.5% or as a linear gradient from 10% to 25%. The crosslinking agent was piperazine diacrylamide (BioRad, Richmond, CA). Gels were stained with Coomassie Brilliant Blue G followed by silver staining (BioRad, Richmond, CA) or were unstained in order to perform an activity analysis by overlaying the gel with soft agar containing indicator FBB-63 cells (Bhunia, 1987).

Disulfide bond assignment. The presence of four cysteine residues in the sequence of PA-1 gives rise to the possibility that two disulfide bonds exist. Since determination of free sulfhydryl residues using Ellman's reagent (DTNB, Sigma) has indicated the absence of free sulfhydryl groups, the conclusion was made that two disulfide bonds are present in PA-1. The identity of the particular cysteine residues involved in these disulfide linkages is set forth hereinafter.

The distribution of lysine residues in the PA-1 sequence is such that a tryptic digest will provide three peptide fragments containing a single cysteine residue. Disulfide bonded peptides produced by tryptic digestion are reduced to give rise to the component fragments. Amino acid analysis of each fragment is compared to calculated values from sequence data to identify the fragment with its tryptic peptide. In this manner, an unambiguous assignment of the disulfide bonding arrangement is possible.

Tryptic cleavage of PA-1 is expected to give rise to three peptide chains and two single amino acids (see also Figure 1) as shown in Table 1:

## TABLE 1

| Tryptic fragment | Sequence* | Comment |
|---|---|---|
| | K | |
| T1 | YYGNGVTCGK | Cys-9 |
| T2 | HSCSVDWGK | Cys-14 |
| T3 | ATTCIINNGAMAWATGGHQGNHK | Cys-24 |
| | C | Cys-44 |

*See Table 4 for abbreviations

Tryptic digestion was done by reaction of 50 ug of purified PA-1 with 1% by weight trypsin (TPCK, Sigma) overnight at 37°C in 50 ul pH 8 ammonium bicarbonate buffer containing 0.1 mM calcium chloride. The reaction mix was applied to a 4.5 x 25 cm C-18 reversed phase column (Vydac, Hesperia, CA). Peaks were detected by monitoring absorbence at 220 nanometers.

Amino acid analysis was performed after overnight vapor phase hydrolysis with 6 N HCl in vacuo at 110°C. PTC amino acid derivatives were produced by reacting with 10% PITC (Pierce) in 70% ethanol. Analysis of PTC amino acids was accomplished with a 0.45 x 15 cm reversed phase C-18 column (Vydac) using a sodium acetate - acetonitrile gradient. Amino acid hydrolysis standards were purchased from Pierce (Rockford, IL).

The crude tryptic digest was applied to a reversed phase HPLC column and peptides were eluted with an acetonitrile gradient. As shown in Table 2, two major peaks were obtained (peaks 7 and 10) and isolated for further examination. Material from each of the two peaks was reduced and the reduction products were separated by reversed phase HPLC. Two peaks were obtained upon reduction of the peptide peak 7, indicating that it had been composed of two peptides linked by a disulfide bond. Results of amino acid analysis showed that the composition of peak 7a matched the calculated composition of peptide T1 and peak 7b similarly matched T2. Further, the unreduced peak 7 agreed with the sum of T1 and T2 as well as with the sum of 7a and 7b. Since T1 and T2 are connected by a disulfide bond, this bond must be between Cys-9 from T1 and Cys-14 from T2.

TABLE 2: Amino Acid Composition of Reduced PA-1 Tryptic Fragments

| Amino Acid | T1 (2-11) | Peak 7-14.36 | T2 (12-20) | Peak 7-16.03 | T3 (21-43) | Peak 10-21.38 |
|---|---|---|---|---|---|---|
| D+N | 1 | 0.8 | 1 | 1.3 | 3 | 2.0 |
| E+Q | 0 | | 0 | | 1 | 1.1 |
| S | 0 | | 2 | 2.1 | 0 | 1.1 |
| G | 3 | 1.7 | 1 | 2.0 | 4 | 5.3 |
| H | 0 | | 1 | | 2 | 1.6 |
| T | 1 | 0.8 | 0 | | 3 | 2.9 |
| A | 0 | | 0 | | 4 | 4.0 |
| Y | 2 | 0.5 | 0 | | 0 | |
| V | 1 | 0.6 | 1 | 1.1 | 0 | 0.9 |
| M | 0 | | 0 | | 1 | 0.4 |
| C | 1 | | 1 | | 1 | |
| I | 0 | | 0 | | 2 | 0.6 |
| K | 1 | 1.0 | 1 | 1.0 | 1 | 1.1 |
| W | 0 | nd | 1 | nd | 1 | nd |

Peak 7-14.36 is most likely T1, 7-16.03 is T2 and 10-21.38 is T3. Disulfide bond assignments: Cys-9 and Cys-14, Cys-24 and Cys-44. nd = not determined.

A second disulfide bond must then exist between Cys-24 and Cys-44. Reduction of this second bond would yield only a single peak in the chromatogram since the peptide bond to Cys-44 was disrupted by tryptic cleavage. Detection at 220 nm is sensitive to peptide bonds, not single amino acids. Peak 10 was reduced and rechromatographed, giving rise to a single peak. Amino acid analysis indicated a strong similarity to tryptic fragment T3. Since fragment T3 contains Cys-24, a disulfide bond to Cys-9 or Cys-14 would have been detected by the appearance of multiple peaks upon reduction of this fragment. Since a single peak was obtained after reduction, Cys-24 is presumed disulfide bonded to Cys-44.

Peptide 7a, corresponding to T1, contains an excess over the one lysine residue predicted. Unreduced peptide 7 also displays this apparent anomaly, with 2.6 moles lysine instead of 2. An explanation of these results is that the N-terminal lysine residue is resistant to the action of trypsin. This is not a really surprising result since the N-terminal is often a freely mobile region although folding might hinder mobility. The N-terminal lysine residue might present difficulty in binding to trypsin due to this mobility. In addition, the amino terminal amino group might be somewhat positively charged at pH 8 and might also interfere with trypsin binding.

Crude bacterial culture medium was applied to the AcA 202 gel filtration resin and bacteriocin activity eluted beginning at the column void volume and continuing for several column volumes. Since the exclusion volume of this gel filtration medium is 20 KDa, the apparent molecular weight of PA-1 activity in crude medium is nearly 20 KDa. This result has also been observed during tangential filtration using a 10 KDa membrane and during hollow fiber diafiltration using 6KDa fiber pore size. In all cases, PA-1 activity in crude medium is associated with an apparent molecular weight of greater than 10 KDa. In a previous gel filtration study, purified PA-1 eluted from a calibrated gel filtration column at an apparent molecular weight of 16,500. After elution from the cation exchange resin and dialyzing, PA-1 activity elutes much later from the AC202 column. Table 3 shows the activity of the various fractions.

## TABLE 3
### Purification of Pediocin PA-1

| Step | Specific Activity | Yield Percent | Fold Purification |
|---|---|---|---|
| Supernatant | 107 | 100 | 1 |
| Gel Filtration | 166 | 50 | 2.2 |
| Ion Exchange | 1330 | 9.4 | 12.5 |
| Dialysis | 6700 | 7.0 | 63 |
| HPLC | 50000 | 0.6 | 470 |

Characterization. An amino acid analysis of PA-1 is shown in Table 4 along with the amino acid content obtained by sequencing. A titration of free cysteine residues with Ellman's reagent (DTNB, Sigma) reveals no free titratable sulfhydryl groups.

## TABLE 4
### Amino Acid Composition of PA-1

| Amino Acid | Measured | Actual | Abbreviations |
|---|---|---|---|
| D+N | 3.8 | 5 | D=Asp |
| E+Q | 1.4 | 1 | N=Asn |
| S | 2.3 | 2 | E=Glu |
| G | 8.0 | 8 | Q=Gln |
| H | 2.7 | 3 | H=His |
| R | 0.3 | 0 | R=Arg |
| T | 3.3 | 4 | T=Thr |
| A | 4.5 | 4 | A=Ala |
| P | 0 | 0 | P=Pro |
| Y | 2.1 | 2 | Y=Tyr |
| V | 2.1 | 2 | V=Val |
| M | 1.4 | 1 | M=Met |
| C | 3.0 | 4 | C=Cys |
| I | 2.3 | 2 | I=Ile |
| L | 0 | 0 | L=Len |
| F | 0.7 | 0 | F=Phe |
| K | 3.8 | 4 | K=Lys |
| Total | 41.7 | 42 | |

Tryptophan not determined.

Disulfide bond assignment.

Separation of PA-1 tryptic fragments by HPLC gave four major peaks. The first eluting peak, when reduced, gave rise to two peaks by HPLC. Amino acid analysis of these two peaks showed strong similarity to the amino acid composition of two tryptic fragments T1 and T2. This is good evidence that a disulfide bond exists between Cys-9 on T1 and Cys-14 on T2. Analysis of free sulfhydryl groups using Ellman's reagent gave a negative result so a second disulfide bond between Cys-24 and Cys-44 is postulated to account for the lack of free sulfhydryl groups.

Sequence analysis. Primary sequence analysis by Chou-Fasman routines indicate a structure as follows: consisting mostly of beta turns and random coil. Residues 21-25 show a propensity for beta sheet.

Example 3

Molecular Weight

The purified bacteriocin is derived from the gene product of plasmid-borne bacteriocin activity expressed in Pediococcus acidilactici strain NRRL-B-18050. A DNA sequence corresponding to the amino acid sequence reported here lies in an open reading frame of a 7.5 MDa plasmid in an area of the plasmid which has been shown necessary for expression of bacteriocin activity. From the sequence data, a molecular weight of 4629 was calculated. This value is lower than the apparent molecular weight calculated by using standard proteins with a mobility versus log molecular weight curve from the SDS gel.

Example 4

Inhibitory Activity of Bacteriocin

The bacteriocin was effective in inhibiting the bacterial strains listed in Table 5.

TABLE 5

| Inhibitory spectrum of purified bacteriocin in PA-1. | |
|---|---|
| Strain | Inhibition |
| Lactobacillus bifermentum 35409 | Positive |
| Lactobacillus casei subsp. casei NCIB 4114 | Positive |
| Lactobacillus casei 842 | Positive |
| Lactobacillus plantarum WSO | Positive |
| Lactobacillus plantarum 8014 | Positive |
| Leuconostoc dextranicum Lde 1.0 | Positive |
| Pediococcus acidilactici 25742 | Positive |
| Pediococcus pentosaceus PMR 25 | Positive |
| Pediococcus spp. (Isolated from radish) | Positive |
| Pediococcus spp. (Isolated from mushroom) | Positive |
| Enterococci (Isolated from cabbage) | Positive |
| Listeria monocytogenes | Positive |
| Lactococcus lactis ML 3 | Negative |

EP 0 453 719 B1

**Claims**
**Claim for the following Contracting States : AT, BE, CH, DE, DK, FR, GB, GR, IT, LI, LU, NL, SE**

1. A peptide purified to homogeneity consisting of an amino acid sequence

```
Lys - Tyr - Tyr - Gly - Asn - Gly - Val - Thr - Cys - Gly -
 1     2     3     4     5     6     7     8     9    10
Lys - His - Ser - Cys - Ser - Val - Asp - Trp - Gly - Lys -
11    12    13    14    15    16    17    18    19    20
Ala - Thr - Thr - Cys - Ile - Ile - Asn - Asn - Gly - Ala -
21    22    23    24    25    26    27    28    29    30
Met - Ala - Trp - Ala - Thr - Gly - Gly - His - Gln - Gly -
31    32    33    34    35    36    37    38    39    40
Asn - His - Lys - Cys
41    42    43    44
```

and disulfide bonds between Cys 9 and Cys 14 and between Cys 24 and Cys 44.

**Claim for the following Contracting State : ES**

1. A method of preparing a peptide consisting of an amino acid sequence

```
Lys - Tyr - Tyr - Gly - Asn - Gly - Val - Thr - Cys - Gly -
 1     2     3     4     5     6     7     8     9    10
Lys - His - Ser - Cys - Ser - Val - Asp - Trp - Gly - Lys -
11    12    13    14    15    16    17    18    19    20
Ala - Thr - Thr - Cys - Ile - Ile - Asn - Asn - Gly - Ala -
21    22    23    24    25    26    27    28    29    30
Met - Ala - Trp - Ala - Thr - Gly - Gly - His - Gln - Gly -
31    32    33    34    35    36    37    38    39    40
Asn - His - Lys - Cys
41    42    43    44
```

and disulfide bonds between Cys 9 and Cys 14 and between Cys 24 and Cys 44 comprising
(i) growing Pediococcus acidilactici strain NRRL-B-18050 in a growth medium to produce said peptide; and
(ii) isolating the peptide in a homogeneous form from said growth medium;
or
(iii) synthesizing said peptide by chemically coupling the individual amino acids in said sequence.

**Patentansprüche**

**Patentanspruch für folgende Vertragsstaaten : AT, BE, CH, DE, DK, FR, GB, GR, IT, LI, LU, NL, SE**

1.   Peptid, zur Homogenität gereinigt und bestehend aus einer Aminosäuresequenz

```
Lys - Tyr - Tyr - Gly - Asn - Gly - Val - Thr - Cys - Gly -
 1     2     3     4     5     6     7     8     9    10
Lys - His - Ser - Cys - Ser - Val - Asp - Trp - Gly - Lys -
11    12    13    14    15    16    17    18    19    20
Ala - Thr - Thr - Cys - Ile - Ile - Asn - Asn - Gly - Ala -
21    22    23    24    25    26    27    28    29    30
Met - Ala - Trp - Ala - Thr - Gly - Gly - His - Gln - Gly -
31    32    33    34    35    36    37    38    39    40
Asn - His - Lys - Cys
41    42    43    44
```

und Disulfidbrücken zwischen Cys 9 und Cys 14 und zwischen Cys 24 und Cys 44.

**Patentanspruch für folgenden Vertragsstaat : ES**

1.   Verfahren zum Herstellen eines Peptids, bestehend aus einer Aminosäuresequenz

```
Lys - Tyr - Tyr - Gly - Asn - Gly - Val - Thr - Cys - Gly -
 1     2     3     4     5     6     7     8     9    10
Lys - His - Ser - Cys - Ser - Val - Asp - Trp - Gly - Lys -
11    12    13    14    15    16    17    18    19    20
Ala - Thr - Thr - Cys - Ile - Ile - Asn - Asn - Gly - Ala -
21    22    23    24    25    26    27    28    29    30
Met - Ala - Trp - Ala - Thr - Gly - Gly - His - Gln - Gly -
31    32    33    34    35    36    37    38    39    40
Asn - His - Lys - Cys
41    42    43    44
```

und Disulfidbrücken zwischen Cys 9 und Cys 14 und zwischen Cys 24 und Cys 44, umfassend
   (i) Kultivieren des Pediococcus acidilactici-Stammes NRRL-B-18050 in einem Kulturmedium, um das besagte Peptid herzustellen; und
   (ii) Isolieren des Peptids in einer homogenen Form aus dem besagten Kulturmedium;
   oder
   (iii) Synthetisieren des besagten Peptids durch chemisches Verknüpfen der einzelnen Aminosäuren in der besagten Sequenz.

**Revendications**
**Revendication pour les Etats contractants suivants : AT, BE, CH, DE, DK, FR, GB, GR, IT, LI, LU, NL, SE**

1. Peptide purifié, jusqu'à homogénéité, consistant en une séquence d'acides aminés

```
Lys - Tyr - Tyr - Gly - Asn - Gly - Val - Thr - Cys - Gly -
 1     2     3     4     5     6     7     8     9     10
Lys - His - Ser - Cys - Ser - Val - Asp - Trp - Gly - Lys -
 11    12    13    14    15    16    17    18    19    20
Ala - Thr - Thr - Cys - Ile - Ile - Asn - Asn - Gly - Ala -
 21    22    23    24    25    26    27    28    29    30
Met - Ala - Trp - Ala - Thr - Gly - Gly - His - Gln - Gly -
 31    32    33    34    35    36    37    38    39    40
Asn - His - Lys - Cys
 41    42    43    44
```

et des ponts disulfures entre Cys 9 et Cys 14 et entre Cys 24 et Cys 44.

**Revendication pour l'Etat contractant suivant : ES**

1. Procédé de préparation d'un peptide consistant en une séquence d'acides aminés

```
Lys - Tyr - Tyr - Gly - Asn - Gly - Val - Thr - Cys - Gly -
 1     2     3     4     5     6     7     8     9     10
Lys - His - Ser - Cys - Ser - Val - Asp - Trp - Gly - Lys -
 11    12    13    14    15    16    17    18    19    20
Ala - Thr - Thr - Cys - Ile - Ile - Asn - Asn - Gly - Ala -
 21    22    23    24    25    26    27    28    29    30
Met - Ala - Trp - Ala - Thr - Gly - Gly - His - Gln - Gly -
 31    32    33    34    35    36    37    38    39    40
Asn - His - Lys - Cys
 41    42    43    44
```

et des ponts disulfures entre Cys 9 et Cys 14 et entre Cys 24 et Cys 44 consistant à :
(i) mettre en culture la souche NRRL-B-18050 de Pediococcus acidilactici dans un milieu de culture pour produire ledit peptide; et
(ii) isoler ledit peptide sous une forme homogène à partir dudit milieu de culture;
ou
(iii) synthétiser ledit peptide par liaison chimique des acides aminés individuels dans ladite séquence.

```
                                   T1
        <-----------------------------------------------------------
Lys  -  Tyr  -  Tyr  -  Gly  -  Asn  -  Gly  -  Val  -  Thr  -  Cys  -  Gly  -

                                   T2
-->        <------------------------------------------------------------>
Lys  -  His  -  Ser  -  Cys  -  Ser  -  Val  -  Asp  -  Trp  -  Gly  -  Lys  -

                                   T3
        <-----------------------------------------------------------
Ala  -  Thr  -  Thr  -  Cys  -  Ile  -  Ile  -  Asn  -  Asn  -  Gly  -  Ala  -


        ------------------------------------------------------------
Met  -  Ala  -  Trp  -  Ala  -  Thr  -  Gly  -  Gly  -  His  -  Gln  -  Gly  -


        --------------->
Asn  -  His  -  Lys  -  Cys
```

## FIG. 1